(19) **Europäisches Patentamt / European Patent Office / Office européen des brevets**

(11) **EP 1 784 161 B1**

(12) **EUROPEAN PATENT SPECIFICATION**

(45) Date of publication and mention of the grant of the patent:
**02.01.2008 Bulletin 2008/01**

(51) Int Cl.:
*A61K 9/00* *(2006.01)*        *A61K 9/28* *(2006.01)*
*A61K 9/20* *(2006.01)*        *A61K 31/18* *(2006.01)*

(21) Application number: **05774341.1**

(22) Date of filing: **12.08.2005**

(86) International application number:
**PCT/PL2005/000052**

(87) International publication number:
**WO 2006/016829 (16.02.2006 Gazette 2006/07)**

(54) **CONTROLLED-RELEASE FORMULATION COMPRISING TAMSULOSIN HYDROCHLORIDE**

FORMULIERUNG MIT KONTROLLIERTER FREISETZUNG ENTHALTEND TAMSULOSIN HYDROCHLORID

FORMULATION À LIBÉRATION CONTRÔLÉE COMPRENANT DE L'HYDROCHLORURE DE TAMSULOSINE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**BA HR YU**

(30) Priority: **12.08.2004 PL 36956604**

(43) Date of publication of application:
**16.05.2007 Bulletin 2007/20**

(73) Proprietor: **INSTYTUT FARMACEUTYCZNY**
**PL-01-793 Warszawa (PL)**

(72) Inventors:
• **WIACKOWSKI, Lech**
  **PL-01-876 Warszawa (PL)**
• **SZELEJEWSKI, Wieslaw**
  **PL-03-980 Warszawa (PL)**
• **ZAREMBA, Andrzej**
  **PL-02-032 Warszawa (PL)**
• **PESTA-DYNDA, Edyta**
  **PL-02-495 Warszawa (PL)**
• **MARCHLEWSKA-CELA, Zofia**
  **PL-05-020 Piastow (PL)**

(74) Representative: **Krzywdzinska, Ewa et al**
**Instytut Farmaceutyczny,**
**ul. Rydygiera 8**
**01-793 Warszawa (PL)**

(56) References cited:
**EP-A- 1 413 294**        **US-A1- 2003 147 950**

**Description**

This invention relates to a solid oral controlled-release formulation comprising tamsulosin hydrochloride.

Tamsulosin, (-)-(R)-5-[2-[[2-(o-ethoxyphenoxy) ethyl] amino]propyl] -2-methoxybenzenesulfonamide monohydrochloride, is a selective, competitive antagonist of type $\alpha$1A post-synaptic adrenergic receptors in the prostate, used to treat the symptoms of benign prostatic hyperplasia.

Due to its pharmacokinetic properties, tamsulosin hydrochloride is administered to the patients in the form of an oral, controlled-release formulation, suitable for a once-daily administration.

The formulation that has been brought into the medical practice under the brand names Flomax®, Omnic® and Harnal®, is a capsule comprising pellets of tamsulosin hydrochloride 0.4 mg with methacrylic acid copolymer and microcrystalline cellulose as the main inactive ingredients.

In the European Patent Applications EP 0533297 A1 and EP 0194838 A1, there is disclosed the composition of particles, from which tamsulosin is gradually released due to the use of a matrix of a macromolecular, water-insoluble release controlling agent selected from acrylic acid polymers and acrylic acid copolymers. This release controlling agent is used as an aqueous suspension or emulsion or as a solution in an aqueous-organic system and acts also as a binder in the granulation step. Microcrystalline cellulose, used as a carrier, constitutes at least 50 % by weight based on the weight of the formulation, and provides, on granulation, the cohesive particles. The particles containing tamsulosin hydrochloride are formed into tablets, capsules or granules. It is believed that the composition disclosed in EP 0533297 A1 covers the commercially available capsules Flomax®.

International Patent publications WO 2004/043448, WO 2004/043449, WO 2004/056354 and WO 03/039530 disclose some other formulations of tamsulosin hydrochloride in the form of pellets and tablets, all of them using the acrylic polymers as release controlling agents.

Although they are frequently used in pharmaceutical preparations as release controlling and film coating agents (Handbook of Pharmaceutical Excipients, 2nd Ed., 1994, p.362-), methacrylic acid polymers and copolymers are recently raising fears concerning safety of mucous membranes of the gastrointestinal system. Therefore, their gradual withdrawing or at least, limiting exposure of patients to these compounds seems to be well grounded.

In the publication of International Patent Application WO 94/06414 the hydrogel-type sustained-release pharmaceutical formulation of tamsulosin hydrochloride that is releasing the active ingredient not only in the upper gastrointestinal tract, but also upper gastrointestinal tract, but also in the lower gastrointensive tract, in particular in colon, has been proposed. The formulation contains a hydrophilic additive allowing for penetration of water into the core of the formulation as well as a hydrogel-forming polymer. Appropriate extent of gelling is achieved by using a hydrophilic substance that is dissolved in the amount of water not exceeding 5 ml (per 1 g of the active ingredient), such as polyvinylpyrrolidone, D-sorbit or PEG 6000, in combination with a hydrogel-forming polymer characterized by average molecular weight over 2,000,000 or by viscosity of a 1% solution not less than 1,000 cPs.

However, neither a hydrogel-type formulation nor any other formulation of tamsulosin hydrochloride in the form of tablets has been brought into medical practice as yet. Therefore, there is still a need for drug formulations being an alternative to pellets that would be simple in manufacturing while having equivalent active ingredient *in vitro* release profile and pharmacological activity.

This objective has been achieved by developing a solid oral controlled-release formulation in the form of enteric-coated tablets, exhibiting a dissolution profile of tamsulosin hydrochloride, as measured in a Type II paddle apparatus in accordance with the dissolution testing method specified in the European Pharmacopoeia at $37 \pm 0.5°C$ and 100 rpm in a 0.1 N HCl buffer for 2 hours, followed by pH 7.2 buffer for the rest of test:

>     10-40% during first 2 hours (in HCl),
>     35-70% after 3 h (in pH 7.2 buffer system),
>     not less than 70% of the declared content after 5 h (in pH 7.2 buffer system).

The present invention relates to an enteric coated tablet formulation for a controlled release of tamsulosin hydrochloride wherein an active substance is homogenously dispersed within a matrix consisting of a mixture of fatty component and hydrophilic component, together with at least one diluent and optionally other pharmaceutically acceptable excipients.

The fatty component of the matrix can be natural or synthetic substances selected from the group consisting of long-chain fatty acids $C_{12}$-$C_{18}$, glycerides of medium- and long-chain fatty acids $C_8$-$C_{18}$; hydrogenated fatty oils such as castor oil; hydrogenated lecithins or the mixtures thereof.

In a preferred embodiment of the invention, the fatty component of the matrix is a glyceride of a fatty acid, such as glycerol palmitostearate, glycerol monostearate, glycerol behenate. The most preferred fatty component of the matrix is glycerol behenate.

Glycerol behenate, such as commercially available Compritol 888 ATO, is a natural product being a mixture of 12-18% of mono-, 52-54% of di- and 28-32% of triglycerides of docosanoic acid (over 87% of the fatty acid fractions). Glycerol behenate and other glycerides of fatty acids are used as tablets and capsules diluents and, in lower concentration, as lubricants. They may also form lipophilic matrixes for sustained release tablet and microsphere formulations.

The content of the fatty component of the matrix in the formulation of the invention is 20-40% by weight based on the weight of the tablet's core.

The hydrophilic component of the matrix can be any pharmaceutically acceptable inert substance that would allow for penetration of water into the tablet's core, thereby swelling, gelling or thickening and forming a viscous layer facilitating the diffusion of an active substance. Suitable components of this type comprise, e.g. polyvinylpyrrolidone, polyvinyl alcohols, polyethylene glycols, ethers and esters of cellulose, preferably hydroxypropylmethylcellulose, sodium carboxymethylcellulose, alginates and the mixtures thereof.

In a preferred embodiment of the invention, the hydrophilic component of the matrix is polyvinylpyrrolidone, in particular polyvinylpyrrolidone of a molecular weight within the range of 25,000 to 300,000. Particularly preferred is polyvinylpyrrolidone of a molecular weight about 50,000. In the formulation according to the invention polyvinylpyrrolidone is also a binding agent.

The content of the hydrophilic component in the tablet's core depends on the type of the substance used. In case of polyvinylpirrolidones, their content depends on their molecular weight and solubility associated therewith as well as on other physical properties. It could be selected by those skilled in the art on the basis of PVP characteristics and a common knowledge.

In case of using polyvinylpyrrolidone of a molecular weight about 50,000, its fraction is preferably 5-12% by weight based on the weight of the tablet's core.

The assumed profile of controlled-release of tamsulosin hydrochloride is achieved at the weight ratio of the fatty and hydrophilic components of the tablet's core within the range from 2:1 to 6:1, preferably at about 4:1.

The core of the formulation according to the invention further comprises at least one diluent that can be any substance increasing the bulk of the tablet.

In the preferred embodiment of the present invention two different diluents of complementary properties are used, one of them facilitating penetration of water into the tablet's core while the other provides its skeleton. Appropriate diluents can be selected from the group consisting of sugar alcohols such as mannitol, sorbitol, xylitol or maltitol; sugars such as glucose, lactose, levulose, sucrose, maltose, glucose and dextrose; starches such as corn starch and potato starch; cellulose derivatives such as microcrystalline cellulose; cellulose acetate; colloidal silica; calcium sulfate; di- and tribasic calcium phosphate; calcium carbonate; non-pareils; talc and other.

Preferred diluents in the formulation according to the invention are the combination of sorbitol and lactose.

Sorbitol, 1,2,3,4,5,6-hexanehexol, is used as a diluent in tablet formulations prepared by either wet granulation or direct compression. Sorbitol, available in an amorphous form as well as in four crystalline polymorphic forms, provides the bulk of the tablet and facilitates the erosion of the core skeleton by water.

Lactose, (O-$\beta$-D-galactopyranosyl-(1$\rightarrow$4)-$\alpha$-D-glucopyranose), that is less soluble in water than sorbitol, is widely used as a filler or diluent in tablets and capsules. It is available in two anomeric forms, $\alpha$ and $\beta$, among which $\beta$-lactose is anhydrous whereas $\alpha$-lactose may be anhydrous or hydrated, having various distribution of molecular weight and flow characteristics. In the formulation according to the invention, lactose monohydrate is preferred due to its highly porous structure and a large specific area.

The core of the tablet according to the invention can further comprise other pharmaceutically acceptable excipients that facilitate the manufacturing process and provide required physical and mechanical properties of the tablet. The additional excipients could be further components of the matrix, such as cellulose derivatives or acrylic polymers; binders such as pregelatinized starch; sodium alginate, polyvinyl alcohol, acacia gum; disintegrants such as methylcellulose, low-substituted hydroxypropylcellulose, sodium carboxymethylcellulose, calcium carboxymethylcellulose, guar gum, crosspovidone, sodium croscarmellose, colloidal silicon dioxide, alginic acid, potassium polyacryline, sodium starch glycolate; hydrophobic agents such as waxes; preservatives; colorants and other substances, as needed.

The present invention relates also to the process for the preparation of the controlled-release formulation of tamsulosin hydrochloride, which process comprises:

(1) wet granulating the blend of the fatty and hydrophillic matrix components, diluent(s) and binder with an aqueous suspension of tamsulosin hydrochloride to form granules;
(2) drying the granules to remove water;
(3) sieving the granules to standarize their size;
(4) admixing the granules with the additional excipients;
(5) compressing the granules mixture into the cores of the tablets; and
(6) coating the cores of the tablets with the acid resistant coating film.

The additional excipients may be added to the composition of a core either before preparing granules containing the active ingredient or to the granules directly before compressing into the tablet cores. In particular, lubricant(s), glidant (s), additional fillers and binders, if any, are being admixed with the dry granules directly before compressing into the tablet cores.

As the glidants colloidal silicon dioxide, magnesium trisilicate, starch, powdered cellulose, tribasic calcium phosphate or talc may be used, which can also play a role of the lubricant. The lubricants can also be calcium stearate, magnesium stearate, zinc stearate, sodium lauryl fumarate, hydrogenated castor oil, hydrogenated vegetable oil and others.

Many of the excipients may play more than one function in the formulation according to the invention.

In general, the additional fillers in the compression process can be the same that are used to form granules or some other substances. Preferably, sorbitol instant is used as the filler with hydrophillic properties.

The content of tamsulosin hydrochloride in the solid oral formulation according to the invention is 0.4 mg or its multiplicity per the unit dosage form.

In the preferred embodiment of the invention, the formulation comprises about 0.2 wt % of tamsulosin hydrochloride, 20-40 wt % of a fatty component and 5-12 wt % of a hydrophilic component of the matrix, based on the weight of the tablet's core, made up to 100% with diluents and other excipients.

In the particularly preferred embodiment of the invention, the core of a tablet contains, in wt % of the core, 0.2 % of tamsulosin hydrochloride, 20-40 % of glycerol behenate, 5-12 % of PVP, 20-40% of lactose, 30-50 % of sorbitol (powder and instant forms combined) and 0.5-1.5 % of magnesium stearate, up to make 100%.

The core of the tablet is protected by a coating resistant to gastric fluids and dissolving only in the neutral to weakly alkaline medium of an intestine. The coatings of this type are described, for example, in Pharmaceutical Dosage Forms and Drug Delivery Systems, H.C. Ansel, I.V. Allen, N.G. Popovich, 7th ed. (1999), Lippincot, Williams&Wilkins. The acid resistant coatings are formed by anionic polymers and copolymers of acrylic acid or methacrylic acid, with (meth)acrylic acid esters, in particular copolymers of methacrylic acid with methyl methacrylate with free acid units; phthalates such as, e.g. cellulose acetate phthalate, cellulose polyacetate phthalate, acetylvinyl polyphthalate, acetylcellulose succinate, copolymers of vinyl acetate and crotonic acid, together with additives such as plasticizers, fillers, dispersing agents, colorants and polishing agents.

The appropriate dissolution profile of tamsulosin hydrochloride in the targeted place of gastrointestinal tract is achieved by covering the cores with copolymers of methacrylic acid and esters thereof, such as those known under the trade name Eudragit, for example, Eudragit S100 or Eudragit L 30 D-55 or the mixtures thereof. Eudragit L 30 D-55 is a water dispersion of a copolymer based on methacrylic acid and acrylic acid ethyl ester with the ratio of free carboxyl groups to the ester app. 1:1. Eudragit S 100 is a copolymer of methacrylic acid and methacrylic acid methyl ester with the ratio of free carboxyl groups to the ester app. 1:2 and it needs to be partially neutralized with aqueous ammonia before coating the cores.

Typically, the coating is 2-12 % by weight of the tablet core.

The solid oral controlled-release formulation of tamsulosin hydrochloride in the form of enteric-coated tablet according to the invention is characterized by appropriate physicochemical parameters and by an adequate release profile of the active ingredient *in vitro.*

The dissolution profile of tamsulosin hydrochloride from the formulation has been measured in a Type II paddle apparatus in accordance with the dissolution testing method specified in the European Pharmacopoeia at $37\pm0.5°C$ and 100 rpm in two steps:.

I. In a 0.1 M solution of hydrochloric acid with 0.003% of Tween; after 2 h the dissolution of the active ingredient should be 10-40% of the declared amount;

II. In a phosphate buffer pH 7.2; the dissolution of the active ingredient should be

after 3 h: 35-70%,

after 5 h: not less than 70% of the declared amount.

The dissolution test is performed using 6 tablets placed in 6 separate vessels. Each tablet is placed in a vessel of the apparatus containing 500 mL of hydrochloric acid (0.1 mol/L) pre-heated to $37\pm0.5°C$. After covering the vessels with lids, the agitator is turned on. After 2 hours of stirring, 20 mL of the solution is taken and the amount of dissolved tamsulosin hydrochloride is determined by HPLC using a UV detector at the wavelength $\lambda=225$ nm. Next, the whole volume of the acid is poured out from the vessels and replaced with 500 mL of a phosphate buffer pH 7.2, pre-heated to $37\pm0.5°C$. In pre-defined time intervals (after 3 and 5 hours) 10 mL samples are taken, each time adjusting volume in each vessel to 500 mL with a phosphate buffer having the same pH and temperature. For each sample, content of dissolved tamsulosin hydrochloride is determined by HPLC using a UV detector at the wavelength $\lambda=225$ nm.

The dissolution profiles of the tablet formulation according to the invention and the reference product Omnic® capsules has been compared for similarity by calculating a similarity factor. In accordance with guidelines of Committee for Proprietary Medicinal Products (Note for Guidance on Quality of Modified Release Products):

$$f_2 = 50 \times \log \left\{ \left[ 1 + \left( \frac{1}{n} \right) \sum_{t=1}^{n} (R_t - T_t)^2 \right]^{-0.5} \times 100 \right\}$$

where:

$f_2$ -     similarity factor;

n -     number of time points;

$R_t$ -     mean percent active ingredient dissolved of the reference product;

$T_t$ -     mean percent active ingredient dissolved of the formulation according to the invention.

The similarity factor $f_2$ for the tablets according to the invention was found to be about 70. According to EMEA guidelines, the dissolution profiles of the tablet formulation according to the invention and the reference product Omnic® capsules are similar.

The solid preparation according to the invention provides appropriate in vitro dissolution profile of tamsulosin hydrochloride.

The invention is illustrated by the following examples that, however, do not limit its scope.

Example 1

| Tablet of tamsulosin hydrochloride á 0.4 mg | |
| --- | --- |
| Composition of the tablet's core (in g/1000 tablets): | |
| Tamsulosin hydrochloride | 4.0 |
| Lactose 200/25 | 500.0 |
| Compritol ATO 888 | 560.0 |
| PVP K30 | 140.0 |
| Sorbitol - powder | 500.0 |
| Sorbitol instant | 276.0 |
| Magnesium stearate | 20.0 |
| Total: | 2000.0 |

The excipients have been sieved, if needed, through a 0.5 mm sieve. Lactose, powdered sorbitol and Compritol ATO 888 were stirred for 4 min. at the speed of a planetary-motion paddle of 300 rpm, until a uniform powders blend was obtained. A suspension for granulation was prepared by dispersing tamsulosin hydrochloride (1 wt% excess with respect to the calculated amount), after sieving it through a 0.3 mm sieve, in water (40 mL per 10,000 tablets). After emptying, the reactor was washed with 50 mL of water that was added to the suspension. The suspension was added to the mixture in the granulator. Granulation has taken 16 min. at 300 rpm of the planetary-motion paddle and 1,500 rpm of the high-speed propeller. After 8 min. of stirring, speed of the propeller was increased to 3,000 rpm. The granules, obtained this way were dried in a fluidized-bed dryer at 30°C for 8 min., to reach moisture content 0.5-2.0 wt%, and then particle size of the granules was standardized using an oscillating granulator provided with a 0.8 mm sieve. After standardization, granules were weighed. Necessary amounts of magnesium stearate and sorbitol instant were calculated on weight of the granules. Weighed amounts of both substances, after careful blending in a barrel-shape blender (15 min., 15 rpm), were added to the granules and the mixture was carefully blended and then compressed on a rotary tableting machine, using 8 mm biconvex punches and controlling the weight of the tablets.

The tablet cores, obtained as above, after determining their weight, hardness, friability and dissolution rate, were de-dusted and coated in a pan coater, pre-heated to app. 40°C. The coating suspension comprising (in mg/500 mg):

| | |
| --- | --- |
| Eudragit S 100 | 60.0 |
| Triacetin | 30.0 |
| Talc | 20.0 |

(continued)

| Lactose | 6.0 |
|---|---|
| Aqueous ammonia | q.s., |

was prepared by dispersing the calculated amounts of metacrylic acid polymer and triethyl citrate (triacetin) with aqueous ammonia with high-speed stirring, to partially neutralize -COOH groups, and after that homogenous suspension of lactose and talc in the remaining volume of water was added.

The coating process was carried out in a pan coater at a temperature of the tablet bed 40°C, until a uniform coating, 10-12 mg per tablet, was obtained. After completion of coating, the tablets were tentatively dried for about 30 min., by slowing down revolutions of the coater's drum and lowering air temperature at the inlet to 40-50°C. The tablets, spread loosely on trays, were dried in a tray drier for 2 h at 40°C.

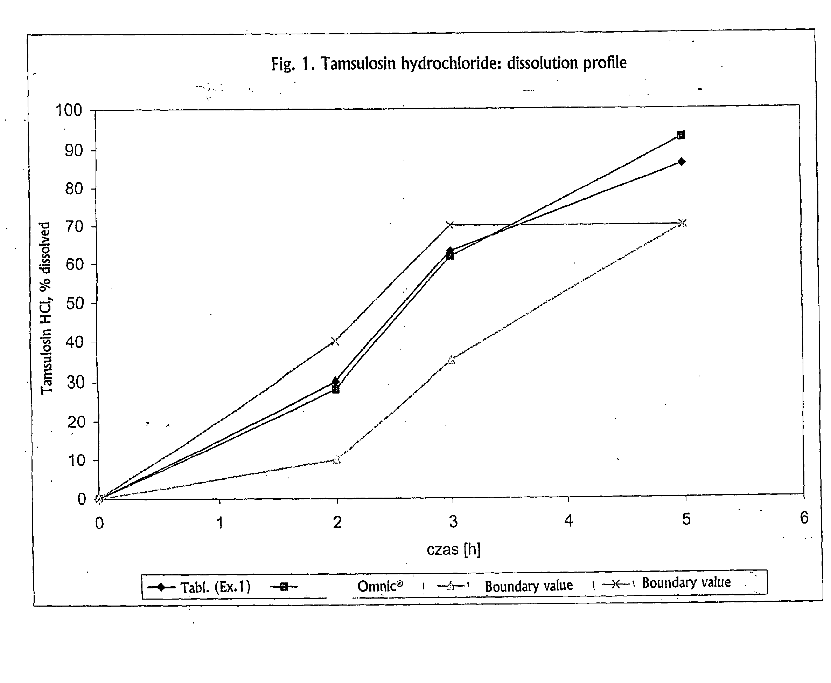

Results of the dissolution test for the formulation of Example 1 and for the reference formulation Omnic® capsules, each comprising 0.4 mg of the active ingredient, are presented in the Table 1 below and on Fig. 1 as diagram representing amount of dissolved tamsulosin hydrochloride (in wt%) *versus* time.

Table 1

| Time [h] | Limits of the dissolved active ingredient [%] | % of the active ingredient released from Omnic® capsules | % of the active ingredient released from the tablets according to the invention |
|---|---|---|---|
| 2 | 10-40 | 28.1 | 30.0 |
| 3 | 35-70 | 61.8 | 63.2 |
| 5 | not less than 70 | 92.9 | 86.2 |

Determination of the dissolution rates of tamsulosin hydrochloride from the tablets prepared as given hereinabove has given a similarity factor $f_2 = 68.7$ with respect to the reference Omnic® capsules.

Stability tests of the tablets prepared as above were run for 3 months at 25 °C, 60% RT, while accelerated ageing was run for 3 months at 40°C, 75% RT. The samples were characterized in terms of physicochemical properties and stability.

Results of the tests are presented in Table 2.

Table 2

| Parameter | Directly after preparation | 3 months | |
|---|---|---|---|
| | | 25°C, 60% RH | 40°C, 75% RH |
| Appearance | White round tablets, biconvex | No changes | No changes |
| Average weight | 200.0 mg | 202.1 mg | 200.7 mg |
| Purity (by HPLC) Any single impurity≤0.1 Sum of impurities < 0.5% | <0.1 <0.5% | <0.1 <0.5% | <0.1 <0.5% |
| Assay of tamsulosin hydrochloride | 0.39 mg | 0.40 mg | 0.41 mg |
| Dissolution rate: - after 2 h - after 3 h - after 5 h | 27.7% 47,5% 84.9% | 23.0% 45.7% 85.1% | 20.9% 49.6% 80.0% |

Example 2

Cores of the tablets obtained as in Example 1 were coated in an analogous manner using a 30 wt % aqueous coating dispersion, containing (in g per 10,000 tablets) :

Eudragit L30 D-55     113.3 g (34.0 g of a solid)

(continued)

| | |
|---|---|
| Triethyl citrate | 3.4 g |
| Talc | 5.0 g |
| Titanium dioxide | 2.5 g |
| Yellow lake | 0.39 g. |

The cores have been placed in the drum of the pan coater and de-dusted. The bed of the cores has been heated up to about 30-34°C with inlet air temperature 45°C and then the cores were coated by a uniform stream of the dispersion. Average weight increase of the coating has been controlled, until it has reached approximately 4.6 mg per tablet. After coating, the tablets have been dried for 5 min. at 35°C (temperature of inlet air).

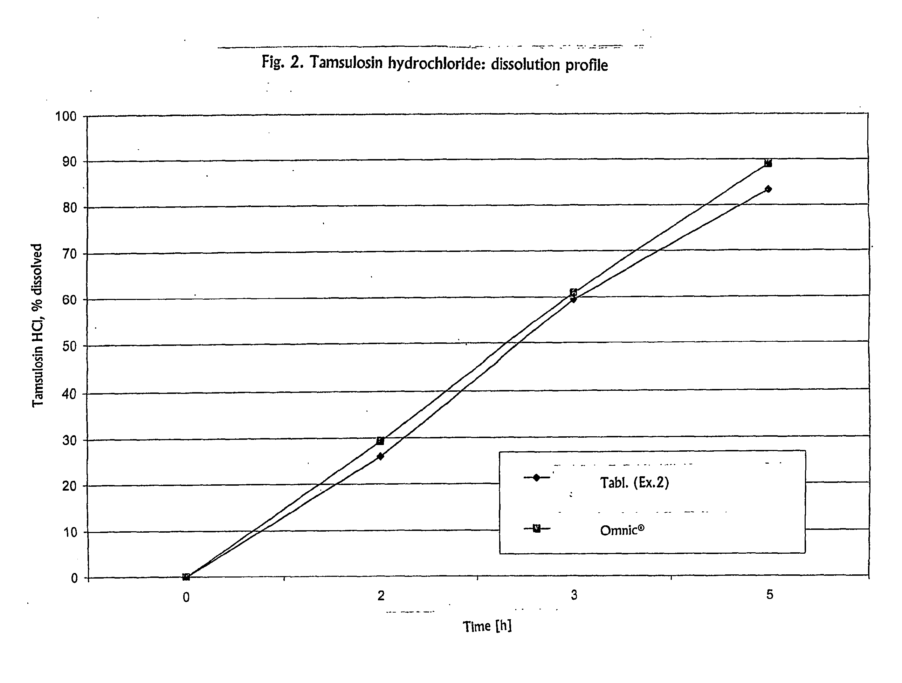

Determination of the dissolution rates of tamsulosin hydrochloride from the tablets prepared as hereinabove has given a similarity coefficient $f_2$ = 70.01 with respect to the reference Omnic® capsules. The dissolution profiles of both formulations are presented on Fig. 2.

## Claims

1. A solid oral controlled-release formulation of tamsulosin hydrochloride in the form of an enteric-coated tablet, wherein tamsulosin hydrochloride is homogenously dispersed within a matrix consisting of a mixture of fatty component and hydrophilic component, together with at least one diluent and optionally other pharmaceutically acceptable excipients.

2. The formulation according to Claim 1, exhibiting a dissolution profile of tamsulosin hydrochloride, as measured in a Type II paddle apparatus in accordance with the dissolution testing method specified in the European Pharmacopoeia at 37±0.5°C and 100 rpm in a 0.1 N HCl buffer for 2 hours, followed by pH 7.2 buffer for the rest of test:

   10-40% during first 2 hours (in HCl),
   35-70% after 3 h (in pH 7.2 buffer system),
   not less than 70% of the declared content after 5 h (in pH 7.2 buffer system).

3. The formulation according to Claims 1-2, wherein the fatty component of the matrix is a fatty acid glyceride.

4. The formulation according to Claim 3, wherein the fatty acid glyceride is selected from the group consisting of long-chain fatty acids $C_{12}$-$C_{18}$, glycerides of medium- and long-chain fatty acids $C_8$-$C_{18}$; hydrogenated fatty oils such as castor oil; hydrogenated lecithins or the mixtures thereof.

5. The formulation according to Claim 4, wherein the fatty acid glyceride is glycerol behenate.

6. The formulation according to Claims 1-5, wherein the weight ratio of the fatty and the hydrophilic component of the matrix is between 2:1 and 6:1.

7. The formulation according to Claim 6, wherein the weight ratio of the fatty and the hydrophilic component of the matrix is about 4:1.

8. The formulation according to Claims 1-7, wherein the hydrophilic component of the matrix is selected from the group consisting of polyvinylpyrrolidone, polyvinyl alcohols, polyethylene glycols, ethers and esters of cellulose, preferably hydroxypropylmethylcellulose, sodium carboxymethylcellulose, alginates and the mixtures thereof.

9. The formulation according to Claim 8, wherein the hydrophilic component of the matrix is polyvinylpyrrolidone.

10. The formulation according to Claim 9, wherein the hydrophilic component of the matrix is particular polyvinylpyrrolidone of a molecular weight within the range of 25,000 to 300,000.

11. The formulation according to Claim 10, wherein the hydrophilic component of the matrix is polyvinylpyrrolidone of a molecular weight about- 50,000.

12. The formulation according to Claims 1-11, wherein the core of the tablet contains two diluents of complementary properties.

13. The formulation according to Claim 12, wherein the diluent is a combination of lactose and sorbitol.

14. The formulation according to Claims 1-13, optionally containing additional matrix component(s), binder(s), lubricant(s), glidant(s), colorants and other pharmaceutically acceptable excipients.

15. The formulation according to Claims 1-14, wherein the content of tamsulosin hydrochloride is 0.4 mg per unit dosage form.

16. The formulation according to Claims 1-15, comprising, in wt% of the tablet core, 0.2% of tamsulosin hydrochloride, 20-40% of glycerol behenate, 5-12% of polyvinylpyrrolidone, 20-40% of lactose, 30-50% of sorbitol and 0.5-1.5% of magnesium stearate, whereas the sum of all constituents equals 100%.

17. The formulation according to Claims 1-16, wherein the coating consists of a copolymer of methacrylic acid.

18. The formulation according to Claim 17, wherein the coating consists of a copolymer of methacrylic acid and acrylic acid ethyl ester.

19. The formulation according to Claims 1-18, wherein the coating is 2-12 % by weight of the tablet's core.

20. A process for the preparation of the controlled-release formulation of tamsulosin hydrochloride, which process comprises:

(1) wet granulating the blend of the fatty and hydrophillic matrix components, diluent(s) and binder with an aqueous suspension of tamsulosin hydrochloride to form granules;
(2) drying the granules to remove water;
(3) sieving the granules to standarize their size;
(4) admixing the granules with the additional excipients;
(5) compressing the granules mixture into the cores of the tablets; and
(6) coating the cores of the tablets with the acid resistant coating film.

21. The process according to Claim 20, in which the additional excipient is sorbitol instant.

**Patentansprüche**

1. Peroral verabreichbare, feste Tamsulosinhydrochloridformulierung von kontrolierter Freisetzung in Form einer magensaftresistenten Tablette, **dadurch gekennzeichnet, dass** Tamsulosinhydrochlorid in einer Matrix, die aus Mischung von einer Fettkomponente und einer hydrophilen Komponente besteht, zusammen mit wenigstens einem Füller und gegebenenfalls anderen pharmazeutisch akzeptablen Exzipienten homogen dispergiert ist.

2. Formulierung nach Anspruch 1, die mit Apparat II (Paddle) nach der in European Pharmacopoeia beschriebenen Testmethode bei $37 \pm 0,5°C$ und 100 Upm in 0,1 N HCl für 2 Std, dann für den Rest vom Test - im Puffer bei pH 7,2 bestimmte Freisetzungsprofil von Tamsulosinhydrochlorid:

10-40% während der ersten 2 Std. (in HCl),
35-70% nach 3 Std. (im Puffer bei pH 7,2),
nicht weniger als 70% der nominalen Gehalt nach 5 Std. (im Puffer bei pH 7,2), aufweist.

3. Formulierung nach Ansprüche 1-2, **dadurch gekennzeichnet, dass** die Fettkomponente in der Matrix Fettsäureglyzerid ist.

4. Formulierung nach Anspruch 3, **dadurch gekennzeichnet, dass** das Fettsäureglyzerid ausgewählt ist aus der Gruppe bestehend aus langkettiger $C_{12}$-$C_{18}$Fettsäuren, Glyzeriden von mittleren und langkettiger $C_8$-$C_{18}$Fettsäuren; hydrierten Fettölen, wie Castoröl; hydrierten Lecithinen oder deren Mischungen.

5. Formulierung nach Anspruch 4, **dadurch gekennzeichnet, dass** das Fettsaureglyzerid Glyzerinbehenat ist.

**6.** Formulierung nach Ansprüche 1-5, **dadurch gekennzeichnet, dass** das Gewichtsverhältnis von der Fettkomponente zur hydrophilen Komponente in der Matrix zwischen 2:1 und 6:1 liegt.

**7.** Formulierung nach Anspruch 6, **dadurch gekennzeichnet, dass** das Gewichtsverhältnis von der Fettkomponente zur hydrophilen Komponente in der Matrix ca. 4:1 beträgt.

**8.** Formulierung nach Ansprüche 1-7, **dadurch gekennzeichnet, dass** die hydrophile Komponente in der Matrix ausgewählt ist aus der Gruppe bestehend aus: Polyvinylpyrrolidon, Polyvinylalkoholen, Polyethyleneglykolen, Celluloseether und -ester, bevorzugt Hydroxypropylmethylcellulose, Natriumcarboxymethyl-cellulose, Alginaten und deren Mischungen.

**9.** Formulierung nach Anspruch 8, **dadurch gekennzeichnet, dass** die hydrophile Komponente in der Matrix Polyvinylpyrrolidon ist.

**10.** Formulierung nach Anspruch 9, **dadurch gekennzeichnet, dass** die hydrophile Komponente in der Matrix ein partikuläres Polyvinylpyrrolidon mit Molekulargewicht im Bereich von 25000-300000 ist.

**11.** Formulierung nach Anspruch 10, **dadurch gekennzeichnet, dass** die hydrophile Komponente in der Matrix Polyvinylpyrrolidon mit Molekulargewicht ca. 50000 ist.

**12.** Formulierung nach Ansprüche 1-11, **dadurch gekennzeichnet, dass** der Tablettekern zwei Füller, die komplementäre Eigenschaften besitzen, enthält.

**13.** Formulierung nach Anspruch 12, **dadurch gekennzeichnet, dass** der Füller eine Kombination von Lactose und Sorbitol ist.

**14.** Formulierung nach Ansprüche 1-13, die gegebenenfalls weitere Matrixkomponente(n), Bindemittel, Lubricant(e), Gleitmittel, Pigmente und andere pharmazeutisch akzeptabel Exzipiente enthalten.

**15.** Formulierung nach Ansprüche 1-14, **dadurch gekennzeichnet, dass** der Anteil an Tamsulosinhydrochlorid 0,4 mg per Dosierungseinheit beträgt.

**16.** Formulierung nach Ansprüche 1-15, derer Tablettenkern, 0,2% Tamsulosinhydrochlorid, 20-40% Glyzerinbehenat, 5-12% Polyvinylpyrrolidon, 20-40% Lactose, 30-50% Sorbitol und 0,5-1,5% Magnesiumstearat, in Gew.-%, enthält, wobei die Summe aller Komponenten 100% ist.

**17.** Formulierung nach Ansprüche 1-16, **dadurch gekennzeichnet, dass** der Überzug aus Metacrylsäure-Copolymer besteht.

**18.** Formulierung nach Anspruch 17, **dadurch gekennzeichnet, dass** der Überzug aus Copolymer von Metacrylsäure und Acrylsäureethylester besteht.

**19.** Formulierung nach Ansprüche 1-18, **dadurch gekennzeichnet, dass** der Überzug 2 bis 12 Gew.-% von Tablettenkern beträgt.

**20.** Verfahren zur Herstellung von Tamsulosinhydrochloridformulierung von kontrollierter Freisetzung, wobei der Verfahren die folgenden Schritte umfasst:

(1) Naßgratulation der Mischung der Fettkomponente und der hydrophilen Komponente der Matrix, Füller(s) und Bindemittel mit wässrigen Suspension von Tamsulosinhydrochlorid um Granulat zu formen;
(2) Trocknen des Granulats um Wasser zu beseitigen;
(3) Sieben des Granulats um seine Korngröße zu standarisieren;
(4) Zumischen von weiteren Exzipienten zum Granulat;
(5) Verpressen der Granulatsmischung zu Tablettenkerne; und
(6) Beschichtung der Tablettenkerne mittels säureresistanter Filmschicht.

**21.** Verfahren nach Anspruch 20, **dadurch gekennzeichnet, dass** ein weiteres Exzipient Sorbitol instant ist.

**Revendications**

1. Formulation solide, destinée à la voie orale à libération contrôlée de chlorhydrate de tamsulosin sous la forme d'un comprimé gastro-résistant, **caractérisée en ce que** chlorhydrate de tamsulosin est dispersé homogénement dans la matrice consistant de mélange d'un composant gras et un composant hydrophile, avec au moins d'un diluent et eventuellement autres excipients pharmaceutiquement acceptables.

2. Formulation selon la revendication 1, qui présente un profil de dissolution de chlorhydrate de tamsulosin, mesuré dans un appareil à palettes de Type II conformément à la methode de test de dissolution selon la méthode décrite dans la Pharmacopée Européenne à $37 \pm 0.5°C$ et 100 rpm en milieu acide chlorhydrique 0,1N pendant 2 heures, puis pendant la reste du test - en milieu tampon pH 7,2:

   10-40% pendant 2 heures initiales (en milieu HCl),
   35-70% après 3 heures (en milieu tampon pH 7,2),
   au moins 70% de la teneur declarée après 5 heures (en milieu tampon pH 7,2).

3. Formulation selon les revendications 1-2,
   **caractérisée en ce que** le composant gras de la matrice est un glycéride d'acide gras.

4. Formulation selon la revendication 3,
   **caractérisée en ce que** le glycéride d'acide gras est choisi dans le groupe comprenant acides gras $C_{12}$-$C_{18}$ de chaine longue, glycérides des acides gras $C_8$-$C_{18}$ de chaine longue et moyenne; huiles grasses hydrogénées comme huile de ricin; lecithins hydrogénés ou ses melanges.

5. Formulation selon la revendication 4,
   **caractérisée en ce que** le glycéride d'acide gras est behenate de glycérol.

6. Formulation selon les revendications 1-5,
   **caractérisée en ce que** la proportion pondérale du composant gras et du composant hydrophile de la matrice est comprise entre 2:1 et 6:1.

7. Formulation selon la revendication 6,
   **caractérisée en ce que** la proportion pondérale du composant gras et du composant hydrophile de la matrice est 4:1 environ.

8. Formulation selon les revendications 1-7,
   **caractérisée en ce que** le composant hydrophile de la matrice est choisi dans le groupe comprenant polyvinylpyrrolidone, alcools de polyvinyle, glycols polyéthylène, éthers et esters de cellulose, de préférence hydroxypropylméthylcellulose, carboxyméthylcellulose de sodium, alginates et ses melanges.

9. Formulation selon la revendication 8,
   **caractérisée en ce que** le composant hydrophile de la matrice est polyvinylpyrrolidone.

10. Formulation selon la revendication 9,
    **caractérisée en ce que** le composant hydrophile de la matrice est polyvinylpyrrolidone particulier de la masse moléculaire dans un intervalle 25000-300000.

11. Formulation selon la revendication 10,
    **caractérisée en ce que** le composant hydrophile de la matrice est polyvinylpyrrolidone de la masse moléculaire 50000 environ.

12. Formulation selon les revendications 1-11,
    **caractérisée en ce que** un noyau du comprimé contient deux matières de charge de proprietés complémentaires.

13. Formulation selon la revendication 12,
    **caractérisée en ce que** la matière de charge est une combinaison de lactose et sorbitol.

14. Formulation selon les revendications 1-13,

éventuellement comprenant composant(s) supplementaires de la matrice , liant(s), lubrifiant(s), glissant(s), colorants et autres excipients pharmaceutiquement acceptables.

**15.** Formulation selon les revendications 1-14,
**caractérisée en ce que** teneur en chlorhydrate de tamsulosin est 0,4 mg per unité de dosage.

**16.** Formulation selon les revendications 1-15, comprenant 0,2% de chlorhydrate de tamsulosin, 20-40% de behenate de glycérol, 5-12% de polyvinylpyrrolidone, 20-40% de lactose, 30-50% de sorbitol et 0,5-1,5% de stearate de magnesium (exprimé en % de poids du noyau de comprimé), la somme de tout composants étant 100%.

**17.** Formulation selon les revendications 1-16,
**caractérisée en ce que** l'enrobage se compose de copolymère d'acide méthacrylique.

**18.** Formulation selon la revendication 17,
**caractérisée en ce que** l'enrobage se compose de copolymère d'acide méthacrylique et ester éthylique d'acide acrylique.

**19.** Formulation selon les revendications 1-18,
**caractérisée en ce que** l'enrobage constitue 2-12 % en poids du noyau de comprimé.

**20.** Procédé de fabrication de la formulation pour la libération contrôlée de chlorhydrate de tamsulosin, comportant des étapes:

(1) granulation mouillée de mélange d'un composant gras et un composant hydrophile, matière de charge(s) et liant avec une suspension aqueuse de chlorhydrate de tamsulosin afin de former un granulé;
(2) séchage du granulé pour éliminer l'eau;
(3) tamisage du granulé pour standariser sa taille du grain;
(4) mélange du granulé avec des excipients supplémentaires;
(5) compression du mélange du granulé pour produire des noyaus de comprimés; et
(6) revêtement de noyaus de comprimés avec une couche d'une pellicule acide-résistante.

**21.** Procedé selon la revendication 20,
**caractérisée en ce que** l'excipient supplementaire est sorbitol instant.

## Fig. 1. Tamsulosin hydrochloride: dissolution profile

Tabl. (Ex.1) ◆ Omnic® Boundary value Boundary value

Fig. 2. Tamsulosin hydrochloride: dissolution profile

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0533297 A1
- EP 0194838 A1
- WO 2004043448 A
- WO 2004043449 A
- WO 2004056354 A
- WO 03039530 A
- WO 9406414 A

**Non-patent literature cited in the description**

- Pharmaceutical Dosage Forms and Drug Delivery Systems. 1999